# FASCICULE DE BREVET EUROPEEN

(11) **EP 0 766 555 B1**
(45) Date de publication et mention de la délivrance du brevet: **23.12.1998**
(21) Numéro de dépôt: 95923414.7
(22) Date de dépôt: 20.06.1995
(51) Int. Cl.: A61K 9/51

(54) **NANOPARTICULES STABILISEES ET FILTRABLES DANS DES CONDITIONS STERILES**
STABILISIERTE UND UNTER STERILEN BEDINGUNGEN FILTRIERBARE NANOPARTIKEL
NANOPARTICLES STABILIZED AND FILTERABLE IN STERILE CONDITIONS

(30) Priorité: 22.06.1994 FR 9407628
(43) Date de publication de la demande: 09.04.1997
(73) Titulaire: RHONE-POULENC RORER S.A., 92160 Antony (FR)
(72) Inventeur: LABORIE, Michel, 75015 Paris (FR); VACUS, Joel, 75013 Paris (FR); VERRECCHIA, Thierry, 94110 Arcueil (FR); BISSERY, Marie-Christine, 94400 Vitry-sur-Seine (FR)
(86) Numéro de dépôt international: FR9500813
(87) Numéro de publication internationale: WO9535101

(56) Documents cités:
- EP-A- 0 167 825
- EP-A- 0 520 888
- EP-A- 0 520 889
- WO-A-90/15593
- WO-A-91/15193
- J. CONT. RELEASE, vol. 30, Avril 1994 AMSTERDAM, pages 83-96, XP 000446236 SCHWARZ C. ET AL 'SOLID LIPID NANOPARTICLES (SLN) FOR CONTROLLED DRUG DELIVERY . I. PRODUCTION, CHARACTERIZATION AND STERILIZATION.'

## Description

La présente invention concerne des nanoparticules de très petites dimensions, qui présentent, outre l'avantage de pouvoir circuler dans le flux sanguin sans problème de dimension au niveau des capillaires, les avantages d'être stabilisées, de pouvoir être filtrées de façon stérile, et de pouvoir être lyophilisées.

Dans les demandes de brevet EP 523 183, EP 520 888 et EP 520 889 ont été décrites des particules sphériques de petites dimensions présentant l'avantage d'être injectables. Cependant les nanoparticules ainsi préparées présentent des diamètres moyens de l'ordre de 50 à 500 nm et ne seraient pas stérilisables par filtration stérilisante sans une perte considérable de rendement, et/ou pas lyophilisables du fait d'une stabilité insuffisante.

Dans Eur. J. Pharm. Biopharm., 39(5), 173-191 (1993) les auteurs ont examiné les technologies actuellement disponibles dans le domaine des nanoparticules destinées à l'industrie pharmaceutique. Il est précisé page 182 que la filtration stérile de suspensions de nanoparticules n'a jamais été décrite.

Dans la demande internationale WO 90/15593 un procédé de préparation de particules monodispersées de principes actifs à faible solubilité est décrit. Lesdites particules peuvent éventuellement comprendre un polymère à titre de protecteur colloïdal qui resterait à la surface de la particule.

Il a maintenant été trouvé, et c'est ce qui fait l'objet de la présente invention, que l'on peut préparer des particules dont le diamètre moyen est à 95 % inférieur à 100 nm, et plus précisément dont le diamètre moyen est compris entre 20 et 75 nm et qui peuvent donc être soumises à une filtration stérile sur des filtres de 0,22 µm sans perte du rendement. Ces particules sont en outre plus stables que celles qui pouvaient être obtenues selon l'art antérieur et peuvent être lyophilisées sans entraîner de phénomène d'agglomération des particules.

Selon l'invention, les nanoparticules comprennent au moins un polymère ou un copolymère hydrophobe, non hydrosoluble et non hydrodispersable, émulsionné dans une solution ou dispersion aqueuse de phospholipides et de sels biliaires.

Selon l'invention un principe actif peut être introduit avec le Polymère ou le copolymère dans les nanoparticules.

Les phospholipides sont choisis à titre d'exemple parmi les phospholipides naturels, synthétiques ou semi-synthétiques ; on utilise plus particulièrement les lécithines (phosphatidylcholine), comme par exemple les lécithines d'oeuf ou de soja purifiées (lécithine E100®, lécithine E80®, Phospholipons® par exemple Phospholipon 90®), la phosphatidyléthanolamine, la phosphatidylsérine, le phosphatidylinositol, le phosphatidylglycérol, la dipalmitoylphosphatidylcholine, la dipalmitoylglycérophosphatidylcholine, la dimyristoylphosphatidylcholine, la distéaroylphosphatidylcholine l'acide phosphatidique ou leurs mélanges.

Selon l'invention les sels biliaires sont par exemple des sels dérivés de l'acide cholique choisis notamment parmi les cholates, les taurocholates, les glycocholates, les déoxycholates, les chénodéoxycholates, les glycodéoxycholates, les taurodéoxycholates les taurochénodéoxycholates, les déhydrocholates ou encore des dérivés de ces sels ou des mélanges. On utilise avantageusement les sels de sodium.

Le polymère ou le copolymère hydrophobe, non hydrosoluble et non hydrodispersable peut être choisi parmi les polymères biocompatibles et biodégradables, par exemple les polymères de l'acide lactique ou glycolique ainsi que leurs copolymères, ou les copolymères polylactique/polyoxyde d'éthylène (ou de propylène) de préférence de poids moléculaire compris entre 1000 et 200000, les polymères de l'acide polyhydroxybutyrique, les polylactones des acides gras contenant au moins 12 atomes de carbone ou les polyanhydrides.

Les nanoparticules selon l'invention sont tout à fait adaptées à une application à des principes actifs hydrophobes. Les principes actifs pouvant être utilisés peuvent être choisis parmi les grandes classes de médicaments destinés à la médecine humaine ou vétérinaire. Ils peuvent être également choisis parmi des principes destinés à l'industrie cosmétique, agro-alimentaire ou parmi les agents de diagnostic.

A titre d'exemple, des principes actifs intéressant l'industrie pharmaceutique peuvent être choisis, à titre non limitatif, parmi les antirhumatismaux, les anti-inflammatoires non stéroïdiens, les analgésiques, les anti-tussifs, les psychotropes, les stéroïdes, les barbituriques, les antimicrobiens, les anti-allergiques, les antiasthmatiques, les antispasmodiques et anti-sécrétoires, les cardiovasculaires et les vasodilatateurs cérébraux, les protecteurs cérébraux et les protecteurs hépatiques les agents thérapeutiques du tractus gastrointestinal, les agents anti-cancéreux ou les agents anti-viraux, les vitamines, les contraceptifs, les vaccins....

Selon l'invention, les nanoparticules peuvent être obtenues par la technique d'évaporation de solvant, à partir d'une solution ou d'une dispersion aqueuse de phospholipides et de sels biliaires dans laquelle est ajoutée une phase organique non miscible comprenant le principe actif et le polymère ou le copolymère hydrophobe, non hydrosoluble et non hydrodispersable. Le mélange est pré-émulsionné puis soumis à une homogénéisation et à l'évaporation du solvant organique pour obtenir une suspension aqueuse de nanoparticules de très petites dimensions.

La mise en oeuvre de ce procédé est décrite plus en détail dans les exemples.

La phase organique non miscible est de préférence choisie parmi les solvants volatils pouvant être de bons solvants du système polymère choisi. Par exemple on choisira des esters comme notamment l'acétate d'éthyle, des solvants chlorés, par exemple le dichlorométhane ou le chloroforme, ou encore parmi les cétones comme la méthyl éthyl cétone.

D'une manière générale le principe actif constitue de préférence environ 25 % en poids par rapport à la quantité de polymère introduite. Cependant cette quantité pourra varier, éventuellement être plus faible ou même aller jusqu'à 50 % en poids par rapport à la quantité de polymère ou de copolymère introduite.

La phase organique non miscible est constituée de telle manière que le principe actif et le polymère ou le copolymère représentent de 0,1 à 7 % en poids, de la solution.

La phase aqueuse constituée d'une solution ou d'une dispersion aqueuse de phospholipides et de sels biliaires comprend avantageusement ces constituants dans une proportion molaire respective de 1/1. Néanmoins cette proportion peut varier de telle manière que le rapport molaire phospholipides par rapport aux sels biliaires soit de 0,1 à 1.

La phase aqueuse est constituée de telle manière que les phospholipides et les sels biliaires représentent au total de 0,2 à 2 % en poids dans la solution.

Les quantités relatives de phase organique et de phase aqueuse sont choisies de telle manière que la phase organique représente 20 à 60 % en volume par rapport à la phase aqueuse.

Les nanoparticules ainsi obtenues peuvent être filtrées sans donner lieu à des problèmes de colmatage et avec de bons rendements. La filtration s'effectue par filtrations en cascades sur filtres de porosités décroissantes, suivies d'une dernière filtration sur filtre de 0,22 µm.

De préférence, après la filtration, la suspension obtenue est lyophilisée en présence d'un ou plusieurs agents cryoprotecteurs. L'agent cryoprotecteur constitue environ 5 % (poids/volume) de la suspension soumise à la lyophilisation.

La solution destinée à la lyophilisation comprend certains additifs comme des composés non ioniques, par exemple un agent cryoprotecteur ou un agent destiné à ajuster l'isotonicité de la solution finale à injecter. Ces agents peuvent être choisis parmi des sucres (glucose, mannitol, sorbitol, saccharose par exemple), des polymères [par exemple dextran (dextran 1500, dextran 40000) polyvinylpyrrolidones injectables, polyéthylèneglycol ...], des acides aminés (par exemple le glycocolle), ou tout autre agent pouvant exercer cette fonction.

Elle peut également contenir un/(des) agent(s) conservateur(s). Le cas échéant, le lyophilisat peut être repris au moment de l'emploi par de l'eau pour préparations injectables. Il est entendu que de telles opérations ne modifient pas la dimension des particules.

Les nanoparticules selon l'invention sont particulièrement intéressantes du fait de leur stabilité. Cette stabilité permet notamment d'obtenir un lyophilisat de bonne qualité dont la remise en solution et/ou en suspension, au cours de l'utilisation, est améliorée et pour lequel la suspension reconstituée contient des particules de diamètre voisin de celui des nanoparticules initiales.

Les nanoparticules selon l'invention peuvent être utilisées pour la préparation de compositions stériles destinées aux domaines pharmaceutiques ou vétérinaires, au domaine cosmétique, agroalimentaire ou aux agents de diagnostics.

Cette technique est particulièrement intéressante du fait qu'elle ouvre la voie à la préparation à l'échelle industrielle de suspensions nanoparticulaires stabilisées et décontaminées, éventuellement chargées de principes actifs ce qui n'était pas possible jusqu'alors.

En outre les nanoparticules stabilisées selon l'invention présentent un avantage considérable dans le cas de certains principes actifs comme par exemple les agents anticancéreux de la classe des taxoïdes. Elles permettent en effet d'accroître l'activité du produit par comparaison aux formulations classiques.

Le produit de l'exemple 6 étudié par voie i.v. chez la souris sur le mélanome B16, s'est montré 2 fois plus actif sous forme de composition nanoparticulaire, par comparaison à une formulation classique dans un mélange polysorbate 80 / ethanol / dextrose 5% dans l'eau (5/5/90 en volumes).

La présente invention concerne également les compositions pharmaceutiques constituées des nanoparticules selon l'invention, éventuellement en association avec un ou plusieurs excipients ou adjuvants compatibles et pharmaceutiquement acceptables. Ces compositions sont de préférence des compositions injectables.

L'administration par voie parentérale comprend les administrations intraveineuse, intrapéritonéale, intramusculaire ou sous-cutanée. Plus particulièrement préférée est l'administration intrapéritonéale ou intraveineuse.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Chez l'homme, les doses sont généralement comprises entre 0,01 et 200 mg/kg. Par voie intraveineuse, les doses sont généralement comprises entre 0,1 et 50 mg/kg, de préférence entre 0,1 et 8 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

Les exemples suivants, donnés à titre non limitatif, illustrent la présente invention.

### Exemple 1

4,0 g de poly(acide d,l-lactique) d'une masse moléculaire de 2000 D et 1,0 g de diphényl-7,7 (méthoxy-2 phényl)-4 [(méthoxy-2 phényl)-2 propionyl-(S)]-2 perhydroisoindolol-4-(3aS,4S,7aS) sont dissous dans un volume de 100 ml d'acétate d'éthyle chauffé au bain-marie à une température de 45°C (solution A). 1,25 g de cholate de sodium et 1,75 g de lécithine E80® sont dispersés sous agitation à l'Ultra-turrax® dans un volume de 500 ml d'eau pour préparation injectable (solution B).

La solution A est pré-émulsionnée dans la solution B pendant 1 minute à l'Ultra-turrax®. La pré-émulsion est ensuite introduite dans un homogénéisateur de type Microfluidizer 110 T® où elle subit 20 passages successifs à une pression d'utilisation de 6 bars et une température de refroidissement de 0°C.

Un volume de 610 ml de l'émulsion est introduit dans un ballon de 2 litres. L'acétate d'éthyle est évaporé au moyen d'un évaporateur rotatif, sous vide + microfuite, à une température de 30°C, pendant environ 45 minutes. Un volume de 450 ml de suspension nanoparticulaire est récupéré et est complété à 500 ml par de l'eau pour préparation injectable.

Sous une pression de 0,5 bars d'azote, la suspension est filtrée successivement sur 4 membranes d'esters de cellulose Millipore®, d'un diamètre de 45 mm et de porosités décroissantes référencées : RAWP 1,2 µm, AAWP 0,8 µm HAWP 0,45 µm et GSWP 0,22 µm.

La suspension filtrée stérile est partagée en 2 parties. L'une est lyophilisée en présence de 5 % poids/volume de glucose, l'autre est lyophilisée en présence de 5 % poids/volume de saccharose.

Le diamètre moyen des particules mesuré par diffusion de lumière sur un appareil Brookhaven®, avant la lyophilisation et après reprise du lyophilisat par le même volume d'eau pour préparations injectables (dans les 2 cas), est d'environ 70 nm.

Le rendement de fabrication, exprimé par le rapport de la concentration en diphényl-7,7 (méthoxy-2 phényl)-4 [(méthoxy-2 phényl)-2 propionyl-(S)]-2 perhydroisoindolol-4-(3aS,4S,7aS) finale après filtration sur la concentration théorique initiale (2 mg/ml), est de 95 %.

La densité optique à 405 nm de la suspension avant et après la dernière filtration sur 0,22 µm est respectivement de 2,20 et de 2,10.

La concentration en diphényl-7,7 (méthoxy-2 phényl)-4 [(méthoxy-2 phényl)-2 propionyl-(S)]-2 perhydroisoindolol-4-(3aS,4S,7aS) avant et après la dernière filtration sur 0,22 µm reste inchangée.

Le diphényl-7,7 (méthoxy-2 phényl)-4 [(méthoxy-2 phényl)-2 propionyl-(S)]-2 perhydroisoindolol-4-(3aS,4S,7aS) peut être préparé comme décrit dans la demande de brevet WO 93/21155.

### Exemple 2

En opérant comme précédement à l'exemple 1, mais à partir de 4,0 g d'un co-polymère dibloc constitué de l'association d'un poly(acide d,l-lactique) de masse 30 kD et d'un polyéthylène glycol de masse 2 kD (PLA-PEG) et de 1,0 g de diphényl-7,7 (méthoxy-2 phényl)-4 [(méthoxy-2 phényl)-2 propionyl-(S)]-2 perhydroisoindolol-4-(3aS,4S,7aS), on obtient une suspension nanoparticulaire.

Sous une pression de 0,5 bars d'azote, la suspension est filtrée successivement sur 4 membranes d'esters de cellulose Millipore® d'un diamètre de 45 mm et de porosités décroissantes référencées : RAWP 1,2µm, AAWP 0,8 µm HAWP 0,45 µm et GSWP 0,22 µm.

La suspension filtrée stérile est lyophilisée en présence de 5 % p/v de glucose.

Le diamètre moyen des particules mesuré par diffusion de lumière sur un appareil Brookhaven®, avant la lyophilisation et après reprise du lyophilisat par le même volume d'eau pour préparations injectables, est d'environ 75 nm.

Le rendement de fabrication, exprimé par le rapport de la concentration en diphényl-7,7 (méthoxy-2 phényl)-4 [(méthoxy-2 phényl)-2 propionyl-(S)]-2 perhydroisoindolol-4-(3aS,4S,7aS) finale après filtration sur la concentration théorique initiale (2 mg/ml), est de 98 %.

La densité optique à 405 nm de la suspension avant et après la dernière filtration sur 0,22 µm est respectivement de 2,7 et de 2,6.

La concentration en diphényl-7,7 (méthoxy-2 phényl)-4 [(méthoxy-2 phényl)-2 propionyl-(S)]-2 perhydroisoindolol-4-(3aS,4S,7aS) avant et après la dernière filtration sur 0,22 µm reste inchangée.

### Exemple 3

160 mg de poly(acide d,l-lactique) d'une masse moléculaire de 70 000 D et 40 mg de diphényl-7,7 (méthoxy-2 phényl)-4 [(méthoxy-2 phényl)-2 propionyl-(S)]-2 perhydroisoindolol-4-(3aS,4S,7aS) sont dissous dans un volume de 4 ml d'acétate d'éthyle chauffé au bain-marie à une température de 45°C (solution A). 100 mg de cholate de sodium et 70 mg de lécithine E80® sont dispersés sous agitation à l'Ultra-turrax dans un volume de 20 ml d'eau pour préparation injectable (solution B).

La solution A est pré-émulsionnée dans la solution B pendant 1 minute à l'Ultra-turrax. La pré-émulsion est ensuite introduite dans un homogénéisateur de type Microfluidizer 110 S® où elle subit 3 minutes de recyclage continu à une pression d'utilisation de 6 bars et une température de refroidissement de 0°C.

Un volume de 30 ml de l'émulsion est introduit dans un ballon de 200 ml. L'acétate d'éthyle est évaporé au moyen d'un évaporateur rotatif, sous vide + microfuite, à une température de 30°C, pendant environ 45 minutes. Un volume de 19 ml de suspension nanoparticulaire est récupéré et est complété à 20 ml par de l'eau pour préparation injectable.

La suspension est filtrée successivement sur 2 membranes d'esters de cellulose Millipore®, d'un diamètre de 25 mm et de porosités décroissantes référencées : HAWP 1,2 µm et SLGS 0,22 µm.

La suspension filtrée stérile est partagée en 2 parties. L'une est lyophilisée en présence de 5 % poids/volume de glucose, l'autre est lyophilisée en présence de 5 % poids/volume de saccharose.

Le diamètre moyen des particules mesuré par diffusion de lumière sur un appareil Brookhaven®, avant la lyophilisation et après reprise du lyophilisat par le même volume d'eau pour préparations injectables (dans les 2 cas), est d'environ 60 nm.

La densité optique à 405 nm de la suspension avant et après la dernière filtration sur 0,22 µm est respectivement de 1,29 et de 1,14.

La concentration en diphényl-7,7 (méthoxy-2 phényl)-4 [(méthoxy-2 phényl)-2 propionyl-(S)]-2 perhydroisoindolol-4-(3aS,4S,7aS) avant et après la dernière filtration sur 0,22 µm reste inchangée.

### Exemple 4

160 mg de poly(acide d,l-lactique) d'une masse moléculaire de 70 000 D et 40 mg de diphényl-7,7 (méthoxy-2 phényl)-4 [(méthoxy-2 phényl)-2 propionyl-(S)]-2 perhydroisoindolol-4-(3aS,4S,7aS) sont dissous dans un volume de 4 ml d'acétate d'éthyle chauffé au bain-marie à une température de 45°C (solution A). 50 mg de cholate de sodium et 70 mg de lécithine E80® sont dispersés sous agitation à l'Ultra-turrax dans un volume de 20 ml d'eau pour préparation injectable (solution B).

La solution A est pré-émulsionnée dans la solution B pendant 1 minute à l'Ultra-turrax®. La pré-émulsion est ensuite introduite dans un homogénéisateur de type Microfluidizer 110 S® où elle subit 3 minutes de recyclage continu à une pression d'utilisation de 6 bars et une température de refroidissement de 0°C.

Un volume de 30 ml de l'émulsion est introduit dans un ballon de 200 ml. L'acétate d'éthyle est évaporé au moyen d'un évaporateur rotatif, sous vide + microfuite, à une température de 30°C, pendant environ 45 minutes. Un volume de 19 ml de suspension nanoparticulaire est récupéré et est complété à 20 ml par de l'eau pour préparation injectable.

La suspension est filtrée successivement sur 2 membranes d'esters de cellulose Millipore®, d'un diamètre de 25 mm et de porosités décroissantes référencées : HAWP 1,2 µm et SLGS 0,22 µm.

La suspension filtrée stérile est partagée en 2 parties. L'une est lyophilisée en présence de 5 % poids/volume de glucose, l'autre est lyophilisée en présence de 5 % poids/volume de saccharose.

Le diamètre moyen des particules mesuré par diffusion de lumière sur un appareil Brookhaven®, avant la lyophilisation et après reprise du lyophilisat par le même volume d'eau pour préparations injectables (dans les 2 cas), est d'environ 70 nm.

La densité optique à 405 nm de la suspension avant et après la dernière filtration sur 0,22 µm est respectivement de 1,88 et de 1,80.

La concentration en diphényl-7,7 (méthoxy-2 phényl)-4 [(méthoxy-2 phényl)-2 propionyl-(S)]-2 perhydroisoindolol-4-(3aS,4S,7aS) avant et après la dernière filtration sur 0,22 µm reste inchangée.

### Exemple 5

400 mg d'un co-polymère dibloc constitué de l'association d'un poly(acide d,l-lactique) de masse 30 kD et d'un polyethylène glycol de masse 2 kD (PLA-PEG) et 100 mg de diphényl-7,7 (méthoxy-2 phényl)-4 [(méthoxy-2 phényl)-2 propionyl-(S)]-2 perhydroisoindolol-4-(3aS,4S,7aS) sont dissous dans un volume de 10 ml d'acétate d'éthyle chauffé au bain-marie à une température de 45°C (solution A). 125 mg de cholate de sodium et 175 mg de lécithine E80® sont dispersés sous agitation à l'Ultra-turrax® dans un volume de 50 ml d'eau pour préparation injectable (solution B).

La solution A est pré-émulsionnée dans la solution B pendant 1 minute à l'Ultra-turrax®. La pré-émulsion est ensuite introduite dans un homogénéisateur de type Microfluidizer 110 S® où elle subit 10 minutes de recyclage continu à une pression d'utilisation de 6 bars et une température de refroidissement de 0°C.

Un volume de 60 ml de l'émulsion est introduit dans un ballon de 200 ml. L'acétate d'éthyle est évaporé au moyen d'un évaporateur rotatif, sous vide + microfuite, à une température de 30°C, pendant environ 45 minutes. Un volume de 45 ml de suspension nanoparticulaire est récupéré et est complété à 50 ml par de l'eau pour préparation injectable.

La suspension est filtrée successivement sur 2 membranes d'esters de cellulose Millipore®, d'un diamètre de 25 mm et de porosités décroissantes référencées: HAWP 1,2 µm et SLGS 0,22 µm.

La suspension filtrée stérile est lyophilisée en présence de 5 % p/v de glucose.

Le diamètre moyen des particules mesuré par diffusion de lumière sur un appareil Brookhaven®, avant la lyophilisation et après reprise du lyophilisat par le même volume d'eau pour préparations injectables, est d'environ 60 nm.

La densité optique à 405 nm de la suspension avant et après la dernière filtration sur 0,22 µm est respectivement de 0,87 et de 0,81.

La concentration en diphényl-7,7 (méthoxy-2 phényl)-4 [(méthoxy-2 phényl)-2 propionyl-(S)]-2 perhydroisoindolol-4-(3aS,4S,7aS) avant et après la dernière filtration sur 0,22 µm reste inchangée.

### Exemple 6

300 mg (15 mg/ml théorique) d'un co-polymère dibloc constitué de l'association d'un poly(acide d,l-lactique) de masse 30 kD et d'un polyethylène glycol de masse 2 kD (PLA-PEG) et 100 mg (5 mg/ml théorique) de tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α sont dissous dans 8 ml d'acétate d'éthyle (solution A). 70 mg de lécithine E80 et 50 mg de cholate de sodium sont dispersés dans 20 ml de solution glucosée à 5 % p/v (solution B). La solution A est émulsionnée dans la solution B par un Ultra-turrax puis la pré-émulsion est ensuite introduite dans un homogénéisateur de type Microfluidizer 110 S® pendant 3 minutes à 10°C. Le volume d'émulsion récupéré est d'environ 30 ml (30 g). L'acétate d'éthyle est éliminé à l'aide d'un évaporateur rotatif sous pression réduite (100 mm de mercure) jusqu'à un volume de suspension d'environ 17 ml (17 g). La suspension est filtrée sur deux filtres en série de porosité décroissante (1,2 µm Minisart NML® + 0,22 µm SLGS®). Deux suspensions identiques ont été préparées dans les mêmes conditions et réunies dans un même flacon. La suspension filtrée est stérile.

Le diamètre moyen des particules mesuré par diffusion de lumière sur un appareil Brookhaven® est d'environ 75 nm.

La densité optique à 405 nm de la suspension avant et après la dernière filtration sur 0,22 µm est 7,2.

Le rendement de fabrication, exprimé par le rapport de la concentration en tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α finale après filtration, sur la concentration théorique initiale (5 mg/ml), est de 94 %.

La concentration en PLA-PEG (calculée par rapport au rendement en tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α) est 14,1 mg/ml.

Le tert-butoxycarbonylamino-3 hydroxy-2 phényl-3 propionate-(2R,3S) de diacétoxy-4α,10β benzoyloxy-2α époxy-5β,20 hydroxy-1β méthylène-7β,8β oxo-9 nor-19 taxène-11 yle-13α peut être préparé comme décrit dans la demande de brevet WO 94/13654.

## Revendications

1. Nanoparticules stabilisées et filtrables dans des conditions stériles caractérisées en ce qu'elles comprennent au moins un polymère ou un copolymère hydrophobe, non hydrosoluble et non hydrodispersable, émulsionné dans une solution de phospholipides et de sels biliaires.

2. Nanoparticules stabilisées et filtrables dans des conditions stériles, selon la revendication 1, caractérisées en ce qu'un principe actif est introduit avec le polymère ou le copolymère.

3. Nanoparticules stabilisées et filtrables dans des conditions stériles, selon la revendication 1, caractérisées en ce que le polymère ou le copolymère hydrophobe, non hydrosoluble et non hydrodispersable est choisi parmi les polymères biocompatibles et biodégradables.

4. Nanoparticules stabilisées et filtrables dans des conditions stériles, selon l'une des revendications 1 ou 2, caractérisées en ce que leur diamètre moyen est à 95 % inférieur à 100 nm.

5. Procédé de préparation de nanoparticules selon l'une des revendications 1 à 4, caractérisée en ce que l'on prépare une solution ou une dispersion aqueuse de phospholipides et de sels biliaires dans laquelle est ajoutée une phase organique non miscible comprenant le polymère ou le copolymère hydrophobe, non hydrosoluble et non hydrodispersable et le cas échéant le principe actif, puis on effectue une pré-émulsion et soumet le mélange à une homogénéisation et à l'évaporation du solvant organique, puis éventuellement filtre la suspension obtenue et éventuellement lyophilise.

6. Utilisation des nanoparticules stabilisées selon l'une des revendications 1 à 4 pour la préparation de compositions stériles après filtration stérilisante.

7. Procédé de préparation de nanoparticules stabilisées selon la revendication 5 caractérisé en ce que la suspension obtenue subit une opération de filtration stérilisantes en cascades, sur filtres de porosités décroissantes.

8. Nanoparticules stabilisées et filtrables dans des conditions stériles, selon l'une des revendications 1 à 4, caractérisées en ce qu'elles sont lyophilisées.

9. Nanoparticules stabilisées et filtrables dans des conditions stériles, selon l'une des revendications 1 à 4, caractérisées en ce qu'elles ont subi des opérations de filtration stérilisante, de lyophilisation et de remise en solution.

10. Composition pharmaceutique constituée de nanoparticules selon la revendication 1 à 4 ou 8 et 9, éventuellement en association avec un ou plusieurs excipients ou adjuvants compatibles et pharmaceutiquement acceptables.

## Claims

1. Stabilized nanoparticles which may be filtered under sterile conditions, characterized in that they comprise at least one hydrophobic, water-insoluble and non-water-dispersible polymer or copolymer emulsified in a solution of phospholipids and bile salts.

2. Stabilized nanoparticles which may be filtered under sterile conditions, according to claim 1, characterized in that an active principle is introduced with the polymer or the copolymer.

3. Stabilized nanoparticles which may be filtered under sterile conditions, according to claim 1, characterized in that the hydrophobic, water-insoluble and non-water-dispersible polymer or copolymer is chosen from biocompatible and biodegradable polymers.

4. Stabilized nanoparticles which may be filtered under sterile conditions, according to either of claims 1 and 2, characterized in that their mean diameter is, to an extent of 95 %, less than 100 nm.

5. Process for the preparation of nanoparticles according to one of claims 1 to 4, characterized in that an aqueous solution or dispersion of phospholipids and bile salts is prepared, to which is added an immiscible organic phase comprising the hydrophobic, water-insoluble and non-water-dispersible polymer or copolymer and, where appropriate, the active principle, and a pre-emulsification is then carried out and the mixture is subjected to a homogenization and to evaporation of the organic solvent, then the suspension obtained is optionally filtered and optionally freeze-dried.

6. Use of the stabilized nanoparticles according to one of claims 1 to 4 for the preparation of compositions which are sterile after sterilizing filtration.

7. Process for the preparation of stabilized nanoparticles according to claim 5, characterized in that the suspension obtained undergoes an operation of sterilizing filtrations in cascades, on filters of decreasing porosities.

8. Stabilized nanoparticles which may be filtered under sterile conditions, according to one of claims 1 to 4, characterized in that they are freeze-dried.

9. Stabilized nanoparticles which may be filtered under sterile conditions, according to one of claims 1 to 4, characterized in that they have undergone operations of sterilizing filtration, of freeze-drying and of redissolving.

10. Pharmaceutical composition consisting of nanoparticles according to claim 1 to 4 or 8 and 9, optionally in combination with one or more compatible and pharmaceutically acceptable excipients or adjuvants.

## Patentansprüche

1. Stabilisierte und unter sterilen Bedingungen filtrierbare Nanopartikel, dadurch gekennzeichnet, daß sie wenigstens ein hydrophobes, nicht wasserlösliches und nicht in Wasser dispergierbares Polymer oder Copolymer umfassen, das in einer Lösung von Phospholipiden und Gallensäuresalzen emulgiert ist.

2. Stabilisierte und unter sterilen Bedingungen filtrierbare Nanopartikel gemäß Anspruch 1, dadurch gekennzeichnet, daß mit dem Polymer oder dem Copolymer ein Wirkstoff eingeführt wird.

3. Stabilisierte und unter sterilen Bedingungen filtrierbare Nanopartikel gemäß Anspruch 1, dadurch gekennzeichnet, daß das hydrophobe, nicht wasserlösliche und nicht in Wasser dispergierbare Polymer oder Copolymer unter den biokompatiblen und biologisch abbaubaren Polymeren ausgewählt ist.

4. Stabilisierte und unter sterilen Bedingungen filtrierbare Nanopartikel gemäß einem der Ansprüche 1 oder 2, dadurch gekennzeichnet, daß ihr mittlerer Durchmesser zu 95% kleiner als 100 nm ist.

5. Verfahren zur Herstellung von Nanopartikeln gemäß einem der Ansprüche 1 bis 4, dadurch gekennzeichnet, daß man eine wäßrige Lösung oder Dispersion von Phospholipiden und Gallensäuresalzen herstellt, zu der eine nicht mischbare organische Phase, die das hydrophobe, nicht wasserlösliche und nicht in Wasser dispergierbare Polymer oder Copolymer und gegebenenfalls den Wirkstoff umfaßt, hinzugefügt wird, man dann eine Voremulsion ausführt und das Gemisch einer Homogenisierung und der Verdampfung des organischen Lösungsmittels unterzieht, dann die erhaltene Suspension gegebenenfalls filtriert und gegebenenfalls lyophilisiert.

6. Verwendung der stabilisierten Nanopartikel gemäß einem der Ansprüche 1 bis 4 zur Herstellung von nach sterilisierender Filtration sterilen Zusammensetzungen.

7. Verfahren zur Herstellung von stabilisierten Nanopartikeln gemäß Anspruch 5, dadurch gekennzeichnet, daß die erhaltene Suspension einem Vorgang von sterilisierenden Filtrationen in Kaskaden über Filter mit abnehmenden Porositäten unterzogen wird.

8. Stabilisierte und unter sterilen Bedingungen filtrierbare Nanopartikel gemäß einem der Ansprüche 1 bis 4, dadurch gekennzeichnet, daß sie lyophilisiert wurden.

9. Stabilisierte und unter sterilen Bedingungen filtrierbare Nanopartikel gemäß einem der Ansprüche 1 bis 4, dadurch gekennzeichnet, daß sie Vorgängen von sterilisierender Filtration, Lyophilisation und Wiederauflösung unterzogen wurden.

10. Pharmazeutische Zusammensetzung, die aus Nanopartikeln gemäß Anspruch 1 bis 4 oder 8 und 9, gegebenenfalls in Verbindung mit einem oder mehreren kompatiblen und pharmazeutisch annehmbaren Exzipienten oder Adjuvantien, besteht.
